(19) Europäisches Patentamt
European Patent Office
Office européen des brevets

(11) EP 2 638 911 A1

(12) EUROPEAN PATENT APPLICATION

(43) Date of publication:
**18.09.2013 Bulletin 2013/38**

(51) Int Cl.:
***A61K 31/704*** (2006.01) ***A61K 31/7068*** (2006.01)

(21) Application number: **12305295.3**

(22) Date of filing: **14.03.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **SANOFI**
**75008 Paris (FR)**

(72) Inventors:
- **Bourrie, Bernard**
  **75008 Paris (FR)**
- **Casellas, Pierre**
  **75008 Paris (FR)**
- **Cosnier-Pucheu, Sylvie**
  **75008 Paris (FR)**
- **Jegham, Samir**
  **75008 Paris (FR)**
- **Perreaut, Pierre**
  **75008 Paris (FR)**

(74) Representative: **Blot, Philippe Robert Emile**
**Cabinet Lavoix**
**2, place d'Estienne d'Orves**
**75441 Paris Cedex 09 (FR)**

# (54) Novel combinations for treating acute myeloid leukaemia or chronic myeloid leukaemia

(57) The present invention relates to combinations of N-[2-(2,1,3-benzothiadiazol-5-ylamino)-6-(2,6-dichlorophenyl) pyrido[2,3-d]pyrimidin-7-yl]-N'-(1,1-dimethylethyl)-urea and cytarabine and their use for treating AML or CML.

EP 2 638 911 A1

## Description

This invention relates to the treatment of leukaemias, in particular myeloid leukaemias.

Leukaemia is a cancerous disease of the bone marrow and the blood. Four types of leukaemia can be distinguished: chronic myeloid leukaemia, acute myeloid leukaemia, chronic lymphoid leukaemia and acute lymphoid leukaemia.

Myeloid leukaemias of the acute type with a rapid progression are called AML or acute myeloid leukaemia. Myeloid leukaemias of the chronic type with a gradual, less aggressive progression are called CML or chronic myeloid leukaemia. These are clonal diseases of the bone marrow characterized by a clonal expansion of myeloid cells which cannot differentiate normally and accumulate in the bone marrow and the blood.

According to a study by the American Cancer Society, it is estimated that 11,930 new cases of AML and 4,500 new cases of CML will be diagnosed in 2006 in the United States. Over the period from 2002 to 2006, the 5 year survival rate is 20.4% for AML and 42.3% for CML (Cancer Facts and Figures 2006, American Cancer Society, www.leukemia-lymphoma.org/).

According to the French-American-British (FAB) classification of 1976, there are 8 subtypes of AML, referred to as M0 to M7, depending on the type of cells from which the leukaemia develops (Bennett et al, 1976, "Proposals for the classification of the acute leukaemias. French-American-British (FAB) co-operative group". Br J Haematol 33 (4): 451-8).

About 95% of patients suffering from CML bear a gene translocation between chromosomes 9 and 22 of the leukaemic cells. This abnormality, known as Philadelphia chromosome (Ph1), causes proliferation and uncontrolled multiplication of all the types of white cells and platelets.

Currently, several drugs are available for the treatment of leukaemias. However, there remains a need for new active therapeutic compounds for the improvement of the strategies for treatment of patients suffering from leukaemia or the development of a treatment alternative to the treatments already known (Plo et al, Mol Pharmacol, 2002, 62: 304-312) .

The product N- [2- (2, 1, 3- benzothiadiazol- 5- ylamino)- 6- (2, 6- dichlorophenyl) pyrido [2, 3- d] pyrimidin- 7- yl]- N'- (1, 1- dimethylethyl)- urea is described in the international application W02007/003765. Its formula is shown below:

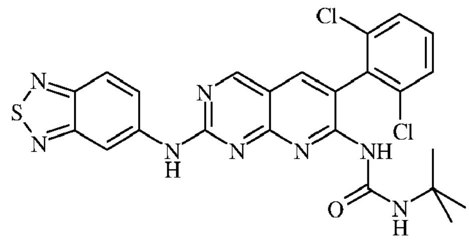

A process for preparation of the compound N- [2- (2, 1, 3- benzothiadiazol- 5- ylamino)- 6- (2, 6- dichlorophenyl) pyrido [2, 3- d] pyrimidin- 7- yl]- N'- (1, 1- dimethylethyl)- urea is also described.

Although this compound can display a significant anti-tumour activity on cells in tests *in vitro,* new parameters such as the distribution of the compound in the tissues, the quantity of product in the serum, the pharmacokinetics and the metabolism are involved in the *in vivo* effect obtained, not predictable on the basis of *in vitro* tests. It has moreover been demonstrated that *in vitro* antitumour activity is not always predictive of *in vivo* activity (Cancer Res. 1988 Oct 1; 48(19): 5447-54, Cancer Chemother Pharmacol. 1996 38: 548-552).

W02008/102075 discloses *in vivo* anti- tumour activity of the compound N- [2- (2, 1, 3- benzothiadiazol- 5-ylamino)- 6- (2, 6- dichlorophenyl) pyrido [2, 3- d] pyrimidin- 7- yl]- N'- (1, 1- dimethylethyl)- urea in animals bearing human leukaemias.

Cytarabine is an anti metabolite drug, mainly used to treat acute leukaemias and non Hodgkin's lymphoma (NHL).

The activity of a combination of the compound N- [2- (2, 1, 3- benzothiadiazol- 5- ylamino)- 6- (2, 6- dichlorophenyl) pyrido [2, 3- d] pyrimidin- 7- yl]- N'- (1, 1- dimethylethyl)- urea with cytarabine for treating AML or CML is however not disclosed in W02008/102075.

The present invention concerns the combination of the compound N- [2- (2, 1, 3- benzothiadiazol- 5- ylamino)- 6- (2, 6- dichlorophenyl) pyrido [2, 3- d] pyrimidin- 7- yl]- N'- (1, 1- dimethylethyl)- urea (A) with cytarabine (B) .

Compound (A) as used herein refers to the compound N- [2- (2, 1, 3- benzothiadiazol- 5- ylamino)- 6- (2, 6-dichlorophenyl) pyrido [2, 3- d] pyrimidin- 7- yl]- N'- (1, 1- dimethylethyl)- urea or a hydrate, a pharmaceutically acceptable salt or a solvate thereof.

Compound (B) as used therein refers to cytarabine.

According to an object, the present invention concerns the use of said combination for treating acute myeloid leukaemia (AML).

According to an object, the present invention concerns the use of said combination for treating chronic myeloid leukaemia (CML).

According to an object, the combinations of the invention are synergistic.

The synergy is herein defined as an effect greater that the added effect of each ingredient.

Said synergy is in particular achieved by the combinations of the invention in inhibiting AML or CML progression, or alleviating AML or CML, more particularly in inhibiting tumor volume and/or tumor weight increase or in reducing tumor volume and/or tumor weight.

According to an embodiment, the compounds of formula (A) and (B) are in amounts that produce a synergistic effect.

The object of the present invention relates to the uses cited above and below for the treatment of mammals, in particular human.

The combinations of the inventions are such that both active ingredients may be administered simultaneously, separately or sequentially.

According to an embodiment, both active ingredients may be administered according to the same administration route or by distinct administration route.

According to an embodiment, both active may be administered in the same dosage form or with separate dosage forms.

Cytarabine is generally administered by the intravenous route (iv) or by intraperitoneal route (ip).

N- [2- (2, 1, 3- benzothiadiazol- 5- ylamino)- 6- (2, 6- dichlorophenyl) pyrido [2, 3- d] pyrimidin- 7- yl]- N'- (1, 1-dimethylethyl)- urea can be administered by the oral route, the intravenous route, the intraperitoneal route, or by two or more routes such as by the intravenous route followed by an intraperitoneal route or by the intravenous route followed by an oral route. In man, a conventional administration route is the intravenous route and/or the oral route. According to the invention, a particular administration route of (A) is the intravenous route followed by the oral route.

In one embodiment, the combination of the invention is for use for treating acute myeloid leukaemia wherein compound (A) is administered by the intravenous route and cytarabine (B) is administered by the intravenous route.

In one embodiment, the combination of the invention is for use for treating acute myeloid leukaemia wherein compound (A) is administered by the oral route and cytarabine (B) is administered by the intravenous route.

In one embodiment, the combination of the invention is for use for treating acute myeloid leukaemia wherein compound (A) is administered by the intravenous route followed by the oral route and cytarabine (B) is administered by the intravenous route.

In one embodiment, the combination of the invention is for use for treating chronic myeloid leukaemia wherein compound (A) is administered by the intravenous route and cytarabine (B) is administered by the intravenous route.

In one embodiment, the combination of the invention is for use for treating chronic myeloid leukaemia wherein compound (A) is administered by the oral route and cytarabine (B) is administered by the intravenous route.

In one embodiment, the combination of the invention is for use for treating chronic myeloid leukaemia wherein compound (A) is administered by the intravenous route followed by the oral route and cytarabine (B) is administered by the intravenous route.

In one embodiment, the combination of the invention is for use for treating chronic myeloid leukaemia wherein compound (A) is administered by the intraperitoneal route followed by the oral route and cytarabine (B) is administered by the intraperitoneal route.

According to an object, the present invention provides for the combination for use for the treatment of AML or CML in patients resistant to standard chemotherapy.

According to another object, the present invention provides for the combination for use for the treatment of AML or CML in high-risk cytogenetic patients.

The expression "high-risk cytogenetic patients" refer to AML or CML patients which display significantly lower rate of response, high-risk of relapse and/or poor survival.

In the present invention, combination is administered according to a dosage scheme which enables the treatment of AML or CML. The dosage scheme varies depending on the administration route and depending on the physical characteristics of the patient. The dosage schemes suitable for this purpose include those which display therapeutic efficacy for the treatment of AML or CML. The combination of the invention can be administered as often as is necessary to obtain the therapeutic effect sought.

The efficacy of the combination of the invention against AML or CML can be determined experimentally as in the following example which illustrates the invention.

The present invention also relates to a kit comprising:

- At least one compound of formula (A),

- Cytarabine (B),
- Instructions for use in the treatment of AML or CML.

The present invention also provides for methods of treatment of AML or CML comprising administration of a combination of the invention to a patient in the need thereof.

The combinations of the invention may be administered in combination with one (or more) anti- cancer active principle (s) , in particular antitumour compounds such as:

- alkylating agents such as the alkylsulphonates (busulfan) , dacarbazine, procarbazine, cloretazine, the nitrogen mustards (chlormethine, melphalan, chlorambucil, cyclophosphamide, ifosfamide) , the nitrosoureas such as carmustine, lomustine, semustine, streptozocine and altretamine;
- antineoplastic alkaloids such as vincristine, vinblastine, vinorelbine and vindesine;
- taxanes such as paclitaxel or taxotere;
- antineoplastic antibiotics such as actinomycin and bleomycin;
- intercalating agents such as mitoxantrone;
- antineoplastic antimetabolites: folate antagonists, methotrexate; inhibitors of purine synthesis; purine analogues such as mercaptopurine and 6-thioguanine; inhibitors of pyrimidine synthesis, aromatase inhibitors, capecitabine, pyrimidine analogues such as fluorouracil, gemcitabine, cytarabine and cytosine arabinoside; brequinar and nelarabine;
- topoisomerase inhibitors such as irinotecan, exatecan, topotecan, teniposide, camptothecin or etoposide;
- anticancer hormone agonists and antagonists including tamoxifen;
- kinase inhibitors such as imatinib, nilotinib and dasatinib, midaustorin, sorafenib, lestaurtinib and tandutinib;
- growth factor inhibitors;
- antiinflammatories such as pentosan polysulphate, corticosteroids, prednisone and dexamethasone;
- ceplene (histamine dihydrochloride);
- anthracyclines such as daunorubicin, epirubicin, pirarubicin, idarubicin, zorubicin, aclarubicin, annamycin, doxorubicin, mitomycin and methramycin;
- anticancer metal complexes, platinum complexes, cisplatin, carboplatin, oxaliplatin and satraplatin;
- alpha interferon,
- triphenylthiophosphoramide;
- antiangiogenic agents;
- thalidomide;
- farnesyl transferase inhibitors such as tipifarnib;
- DNA methyl transferase inhibitors such as MG98;
- immunotherapy adjuvants such as gemtuzumab ozogamicin and HuM 195;
- biotherapeutic agents such as CT388-IL3;
- antisense agents such as GTI-2040;
- vaccines.

The combinations of the invention may also be administered in combination with one or more other active principles useful in one of the pathologies mentioned above, for example an anti-emetic, analgesic, anti-inflammatory or anti-cachexia agent.

It is also possible to combine the compounds of the present invention with a radiation treatment.

These treatments can be administered simultaneously, separately, sequentially. The treatment will be adapted by the doctor depending on the patient to be treated.

A "pharmaceutically acceptable salt" of the compound refers to a salt that is pharmaceutically acceptable and that retains pharmacological activity. It is understood that the pharmaceutically acceptable salts are non-toxic. Additional information on suitable pharmaceutically acceptable salts can be found in Remington's Pharmaceutical Sciences, 17th ed., Mack Publishing Company, Easton, PA, 1985, or S. M. Berge, et al., "Pharmaceutical Salts," J. Pharm. Sci., 1977; 66:1-19, both of which are incorporated herein by reference.

Examples of pharmaceutically acceptable acid addition salts include those formed with inorganic acids such as hydrochloric acid, hydrobromic acid, sulfuric acid, nitric acid, phosphoric acid, as well as those salts formed with organic acids, such as acetic acid, trifluoroacetic acid, propionic acid, hexanoic acid, cyclopentanepropionic acid, glycolic acid, pyruvic acid, lactic acid, oxalic acid, maleic acid, malonic acid, succinic acid, fumaric acid, tartaric acid, citric acid, benzoic acid, cinnamic acid, 3- (4- hydroxybenzoyl) benzoic acid, mandelic acid, methanesulfonic acid, ethanesulfonic acid, 1, 2- ethanedisulfonic acid, 2- hydroxyethanesulfonic acid, benzenesulfonic acid, 4- chlorobenzenesulfonic acid, 2- naphthalenesulfonic acid, 4- toluenesulfonic acid, camphorsulfonic acid, glucoheptonic acid, 4, 4'- methylenebis- (3- hydroxy- 2- ene- l- carboxylic acid) , 3- phenylpropionic acid, trimethylacetic acid, tertiary butylacetic acid, lauryl sulfuric

acid, gluconic acid, glutamic acid, hydroxynaphthoic acid, salicylic acid, stearic acid, muconic acid, p- toluenesulfonic acid, and salicylic acid.

Simultaneous administration typically means that both compounds enter the patient at precisely the same time. However, simultaneous administration also includes the possibility that the compounds enter the patient at different times, but the difference in time is sufficiently miniscule that the first administered compound is not provided the time to take effect on the patient before entry of the second administered compound. Such delayed times typically correspond to less than 1 minute, and more typically, less than 30 seconds. In one example, wherein the compounds are in solution, simultaneous administration can be achieved by administering a solution containing the combination of compounds. In another example, simultaneous administration of separate solutions, one of which contains the compound (A) and the other of which contains cytarabine (B) can be employed. In one example wherein the compounds are in solid form, simultaneous administration can be achieved by administering a composition containing the combination of compounds.

In other embodiments, the compounds are not simultaneously administered. In this regard, the first administered compound is provided time to take effect on the patient before the second administered compound is administered. Generally, the difference in time does not extend beyond the time for the first administered compound to complete its effect in the patient, or beyond the time the first administered compound is completely or substantially eliminated or deactivated in the patient. In one set of embodiments, the compound (A) is administered before cytarabine (B). In another set of embodiments, cytarabine (B) is administered before the compound (A). The time difference in non-simultaneous administrations is typically greater than 1 minute, and can be, for example, precisely, at least, up to, or less than 5 minutes, 10 minutes, 15 minutes, 30 minutes, 45 minutes, 60 minutes, two hours, three hours, six hours, nine hours, 12 hours, 24 hours, 36 hours, or 48 hours, or more than 48 hours.

In one set of embodiments, one or both of compounds are administered in a therapeutically effective (i.e., therapeutic) amount or dosage. A "therapeutically effective amount" is an amount of the active ingredient that, when administered to a patient by itself, effectively achieves at least partially the treatment of AML or CML (for example, inhibits tumor growth, stops tumor growth, or causes tumor regression). An amount that proves "therapeutically effective amount" in a given instance, for a particular subject, may not be effective for 100% of subjects similarly treated for the disease or condition under consideration, even though such dosage is deemed a "therapeutically effective amount" by skilled practitioners. The amount of the compound that corresponds to a therapeutically effective amount is strongly dependent on the type and stage of AML/CML, the age of the patient being treated, and other facts. In general, therapeutically effective amounts of these compounds are well-known in the art, such as provided in the supporting references cited above.

In another set of embodiments, one or both of the active ingredients are administered in a sub-therapeutically effective amount or dosage. A sub-therapeutically effective amount is an amount that, when administered to a patient by itself, does not completely inhibit over time the biological activity of the intended target.

Whether administered in therapeutic or sub-therapeutic amounts, the combination of the invention should be effective in treating AML or CML. A sub-therapeutic amount of compound (A) can be an effective amount if, when combined with cytarabine (B), the combination is effective in the treatment of AML or CML.

In some embodiments, the combination of compounds exhibits a synergistic effect (i.e., greater than additive effect) in treating AML or CML, particularly in reducing a tumor volume and/or weight in the patient. In different embodiments, depending on the effective amounts used, the combination can either inhibit tumor growth and/or weight, achieve tumor stasis, or even achieve substantial or complete tumor regression.

In some embodiments, as shown in the examples, Compound (A) can be administered at a dosage of about 5 mg/kg to 150 mg/kg daily in mice, in particular 10 to 50 mg/kg daily, more particularly 20 mg/kg. Cytarabine, meanwhile, can be administered in mice at a dosage of about 1 mg/kg to 250 mg/kg daily, more particularly about 31,5 mg/kg. Corresponding doses in human can be obtained accordingly. In particular, a typical dosage of cytarabine (B) in human is 2 to 6 mg/kg/day as a continuous IV infusion over 24 hours or in divided doses by rapid injection for 5 to 10 days. Compound (A) may be administered in human at doses comprised between 0.01 mg/g and 1000 mg/kg daily, typically between 50-200 mg/$m^2$. There may be special cases where higher or lower dosages are appropriate; such dosages do not fall outside the scope of the invention. According to the normal practice, the dosage appropriate for each patient is determined by the doctor depending on the mode of administration, and the weight and/or response of the said patient.

The dosage regimen of each active ingredient may be one, two, three or four administration a day or a continuous infusion over time.

As used herein, the term "about" generally indicates a possible variation of no more than 10%, 5%, or 1% of a value. For example, "about 25 mg/kg" will generally indicate, in its broadest sense, a value of 22.5-27.5 mg/kg, i.e., 25 $\pm$ 2.5 mg/kg.

While the amounts of active ingredients should result in the effective treatment of AML or CML, the amounts, when combined, are preferably not excessively toxic to the patient (i.e., the amounts are preferably within toxicity limits as established by medical guidelines). In some embodiments, either to prevent excessive toxicity and/or provide a more efficacious treatment of AML or CML, a limitation on the total administered dosage is provided. Typically, the amounts

considered herein for example are per day; however, half-day and two-day or three-day cycles also are considered herein.

Different dosage regimens may be used to treat AML or CML. In some embodiments, a daily dosage, such as any of the exemplary dosages described above, is administered once, twice, three times, or four times a day for at least three, four, five, six, seven, eight, nine, or ten days. Depending on the stage and severity of the leukaemia, a shorter treatment time (e.g., up to five days) may be employed along with a high dosage, or a longer treatment time (e.g., ten or more days, or weeks, or a month, or longer) may be employed along with a low dosage. In some embodiments, a once- or twice-daily dosage is administered every other day. In some embodiments, each dosage contains both the compound (A) and cytarabine (B), while in other embodiments, each dosage contains either the compound (A) or cytarabine (B). In yet other embodiments, some of the dosages contain both compound (A) and cytarabine (B), while other dosages contain only compound (A) or cytarabine (B).

The patient considered herein is typically a human. However, the patient can be any mammal for which AML or CML treatment is desired. Thus, the methods described herein can be applied to both human and veterinary applications.

The term "treating" or "treatment", as used herein, indicates that the method has, at the least, mitigated abnormal cellular proliferation. For example, the method can reduce the rate of tumor growth in a patient, or prevent the continued growth of a tumor, or even reduce the size and/or weight of a tumor.

In another aspect, methods for preventing AML or CML in a patient are provided. In this regard, prevention denotes causing the clinical symptoms of the disease not to develop in a patient that may be exposed to or predisposed to the disease but does not yet experience or display symptoms of the disease. The methods comprise administering to the patient a combination as described herein. The methods comprise administering to the patient in need thereof a combination, as described herein.

Compounds (A) and (B), or their pharmaceutically acceptable salts or solvate forms, in pure form or in an appropriate pharmaceutical composition, can be administered via any of the accepted modes of administration or agents known in the art. The compounds can be administered, for example, orally, nasally, parenterally (intravenous, intramuscular, or subcutaneous), topically, transdermally, intravaginally, intravesically, intracisternally, or rectally. The dosage form can be, for example, a solid, semi-solid, lyophilized powder, or liquid dosage forms, such as for example, tablets, pills, soft elastic or hard gelatin capsules, powders, solutions, suspensions, suppositories, aerosols, or the like, preferably in unit dosage forms suitable for simple administration of precise dosages. A particular route of administration is oral, particularly one in which a convenient daily dosage regimen can be adjusted according to the degree of severity of the disease to be treated.

The active ingredients or the combination thereof may be in the form of a solid (e.g., a powder or tablet) or a liquid dosage form. The compositions may include optionally, one or more auxiliary (e.g., adjuvant) and/or one or more pharmaceutically acceptable carriers (i.e., vehicles or excipients) known in the art. The said excipients are selected depending on the desired pharmaceutical form and mode of administration, from the normal excipients which are known to the person skilled in the art. Auxiliary and adjuvant agents may include, for example, preserving, wetting, suspending, sweetening, flavoring, perfuming, emulsifying, and dispensing agents. Prevention of the action of microorganisms is generally provided by various antibacterial and antifungal agents, such as, parabens, chlorobutanol, phenol, sorbic acid, and the like. Isotonic agents, such as sugars, sodium chloride, and the like, may also be included. Prolonged absorption of an injectable pharmaceutical form can be brought about by the use of agents delaying absorption, for example, aluminum monostearate and gelatin. The auxiliary agents also can include wetting agents, emulsifying agents, pH buffering agents, and antioxidants, such as, for example, citric acid, sorbitan monolaurate, triethanolamine oleate, butylated hydroxytoluene, and the like.

Dosage forms suitable for parenteral injection may comprise physiologically acceptable sterile aqueous or non-aqueous solutions, dispersions, suspensions or emulsions, and sterile powders for reconstitution into sterile injectable solutions or dispersions. Examples of suitable aqueous and non-aqueous carriers, diluents, solvents or vehicles include water, ethanol, polyols (propyleneglycol, polyethyleneglycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions and by the use of surfactants.

Solid dosage forms for oral administration include soft or hard capsules, tablets, pills, powders, and granules. In such solid dosage forms, the active compound is admixed with at least one inert customary excipient (or carrier) such as sodium citrate or dicalcium phosphate or (a) fillers or extenders, as for example, starches, lactose, sucrose, glucose, mannitol, and silicic acid, (b) binders, as for example, cellulose derivatives, starch, alignates, gelatin, polyvinylpyrrolidone, sucrose, and gum acacia, (c) humectants, as for example, glycerol, (d) disintegrating agents, as for example, agar-agar, calcium carbonate, potato or tapioca starch, alginic acid, croscarmellose sodium, complex silicates, and sodium carbonate, (e) solution retarders, as for example paraffin, (f) absorption accelerators, as for example, quaternary ammonium compounds, (g) wetting agents, as for example, cetyl alcohol, and glycerol monostearate, magnesium stearate and the like (h) adsorbents, as for example, kaolin and bentonite, and (i) lubricants, as for example, talc, calcium stearate,

magnesium stearate, solid polyethylene glycols, sodium lauryl sulfate, or mixtures thereof. In the case of capsules, tablets, and pills, the dosage forms also may comprise buffering agents.

Solid dosage forms as described above can be prepared with coatings and shells, such as enteric coatings and others well-known in the art. They can contain pacifying agents and can be of such composition that they release the active compound or compounds in a certain part of the intestinal tract in a delayed manner. Examples of embedded compositions that can be used are polymeric substances and waxes. The active compounds also can be in microencapsulated form, if appropriate, with one or more of the above-mentioned excipients.

Liquid dosage forms for oral administration include pharmaceutically acceptable emulsions, solutions, suspensions, syrups, and elixirs. Such dosage forms are prepared, for example, by dissolving, dispersing, etc., an active ingredient described herein, or a pharmaceutically acceptable salt thereof, and optional pharmaceutical adjuvants in a carrier, such as, for example, water, saline, aqueous dextrose, glycerol, ethanol and the like; solubilizing agents and emulsifiers, as for example, ethyl alcohol, isopropyl alcohol, ethyl carbonate, ethyl acetate, benzyl alcohol, benzyl benzoate, propyleneglycol, 1, 3- butyleneglycol, dimethyl formamide; oils, in particular, cottonseed oil, groundnut oil, corn germ oil, olive oil, castor oil and sesame oil, glycerol, tetrahydrofurfuryl alcohol, polyethyleneglycols and fatty acid esters of sorbitan; or mixtures of these substances, and the like, to thereby form a solution or suspension.

Suspensions, in addition to the active compounds, may contain suspending agents, as for example, ethoxylated isostearyl alcohols, polyoxyethylene sorbitol and sorbitan esters, microcrystalline cellulose, aluminum metahydroxide, bentonite, agar- agar and tragacanth, or mixtures of these substances, and the like.

Compositions for rectal administrations are, for example, suppositories that can be prepared by mixing the compounds described herein with, for example, suitable nonirritating excipients or carriers such as cocoa butter, polyethyleneglycol or a suppository wax, which are solid at ordinary temperatures but liquid at body temperature and therefore, melt while in a suitable body cavity and release the active component therein.

Dosage forms for topical administration may include, for example, ointments, powders, sprays, and inhalants. The active component is admixed under sterile conditions with a physiologically acceptable carrier and any preservatives, buffers, or propellants as can be required. Ophthalmic formulations, eye ointments, powders, and solutions also can be employed.

Generally, depending on the intended mode of administration, the pharmaceutically acceptable compositions will contain about 1% to about 99% by weight of the compounds described herein, or a pharmaceutically acceptable salt thereof, and 99% to 1% by weight of a pharmaceutically acceptable excipient. In one example, the composition will be between about 5% and about 75% by weight of a compounds described herein, or a pharmaceutically acceptable salt thereof, with the rest being suitable pharmaceutical excipients.

Actual methods of preparing such dosage forms are known, or will be apparent, to those skilled in this art. Reference is made, for example, to Remington’s Pharmaceutical Sciences, 18th Ed., (Mack Publishing Company, Easton, Pa., 1990).

A PEG400 22% / Solutol 5% / G5 73% formulation may be used for the administration by intravenous route of compound (A).

A Labrasol 21% / Solutol 5% / HCl 0.001 N 74% formulation may be used for the administration by the oral route of compound (A).

Kits according to the invention include package(s) comprising combinations of the invention. The phrase "package" means any vessel containing compounds or compositions presented herein. In some embodiments, the package can be a box or wrapping. Packaging materials for use in packaging pharmaceutical products are well-known to those of skill in the art. Examples of pharmaceutical packaging materials include, but are not limited to, bottles, tubes, inhalers, pumps, bags, vials, containers, syringes, bottles, and any packaging material suitable for a selected formulation and intended mode of administration and treatment.

The kit also can contain items that are not contained within the package but are attached to the outside of the package, for example, pipettes.

Kits can contain instructions for administering compounds or compositions of the invention to a patient. Kits also can comprise instructions for approved uses of compounds herein by regulatory agencies, such as the United States Food and Drug Administration. Kits also can contain labeling or product inserts for the inventive compounds. The package (s) and/or any product insert(s) may themselves be approved by regulatory agencies. The kits can include compounds in the solid phase or in a liquid phase (such as buffers provided) in a package. The kits also can include buffers for preparing solutions for conducting the methods, and pipettes for transferring liquids from one container to another.

The term "tumor" as used therein is understood to refer to solid and/or liquid tumors.

The detailed description and specific examples are given for illustration only since various changes and modifications within the spirit and scope of the invention will become apparent to those skilled in the art from this detailed description. Further, the examples demonstrate the principle of the invention and cannot be expected to specifically illustrate the application of this invention to all the examples where it will be obviously useful to those skilled in the prior art.

## BRIEF DESCRIPTION OF THE DRAWINGS

**Figure 1** illustrates the evolution of the median tumor weight (mg) following post tumor implantation in mice implanted with AML KG1 cells and treated with the combinations of the invention.
**Figure 2** illustrates the evolution of the median tumor weight (mg) following post tumor implantation in mice implanted with CML T1 cells and treated with the combinations of the invention.

Examples have been set forth below for the purpose of illustration and to describe certain specific embodiments of the invention. However, the scope of the claims is not to be in any way limited by the examples set forth herein.

## EXAMPLES

### EXAMPLE 1 : Activity on AML

#### Materials and methods

#### Cell lines and primary AML cells

Hematological malignant cell lines were obtained from Deutsche Sammlung von Mikroorganismen und Zellkulturen GmbH (Braunschweig) and cultured in complete RPMI-1640 medium containing 10% fetal bovine serum and antibiotics. Primary AML cells were obtained from patients seen at Toulouse Hospital (Toulouse, France) at the time of diagnosis and after written informed consent. All samples were evaluated for karyotypic abnormalities, immunologic phenotype, FLT3-acquired activating mutations (internal tandem duplications and kinase domain mutations), t (Mizuki M. et al., Blood 2000 96: 3907—3914; Fiebig H.H. et al., Eur. J. Cancer 2004, 40: 802-820) and inv (Bonnet D. et al., Nat Med 1997 3: 730—737) mutations, and c-kit receptor-acquired mutations. Samples were stored in the Hemopathies Inserm Midi-Pyrénées (HIMIP) collection. Mononuclear cells were separated through a Ficoll-Hypaque density gradient.

#### Reagents

For *in vitro* studies, compound (A) was dissolved at 10 mM in DMSO and diluted in complete RPMI-1640 medium or in phenol red-free complete RPMI-1640 medium in sterile 96-well polystyrene cell culture plates. For *in vivo* studies, compound (A) was prepared for intravenous (IV) or intraperitoneal (IP) administration by mixing 22% PEG400 with 5% solutol and 73% glucose in 5% water. For oral administration, compound (A), was prepared in a generally regarded as safe (GRAS) formulation composed of 21% labrasol with 5% solutol and 74% HCL 0.001 N. Solutions were kept on ice and administered as a bolus within 1 hour after formulation preparation. The volume of injection was 0.2 mL per mouse.

#### Myeloid leukemic cell clonogenic assay

The assay is as described by Récher and colleagues (Récher C. et al., Blood 2005; 105:2527-2534) with slight modifications. Briefly, cells were washed twice in PBS and suspended at 1 x $10^5$ cell/mL in H4230 medium (Stem Cell Technologies) supplemented with 10% 5637-conditioned medium and appropriate dilutions of COMPOUND (A). Cells were then plated on 35 mm petri dishes and incubated at 37°C in 5% $CO_2$, fully humidified atmosphere. After 7 days, colonies (more than 20 cells) and clusters (more than 5 cells) were scored using an inverted microscope.

#### Clonogenic assay with human tumor xenografts

Tumor xenografts were derived from patients' tumors engrafted subcutaneously in nude mice (Oncotest). Details of the clonogenic assay procedure have been described earlier (Fiebig H.H. et al., Eur. J. Cancer 2004, 40: 802-820).

#### Normal myeloid progenitor cell clonogenic assay

Fresh $CD34^+$ human bone marrow cells were washed twice in Iscove modified Dulbecco medium (IMDM) containing 10% FCS and resuspended in H4230 medium supplemented with 10% 5637-conditioned medium for CFU-GM growth, in H4435 medium for CFU-M growth, and in H4535 medium for BFU-E growth. H4230, H4435, and H4535 medium were purchased from Stem Cell Technologies. The cells were then plated in 35-mm Petri dishes and incubated

in a humidified $CO_2$ incubator (5% $CO_2$, 37°C) for 14 days. Colonies (>50 cells) were then scored under an inverted microscope.

**Tumor implantation studies in SCID mice**

Eight- week- old female SCID mice were purchased from Charles River. The Committee of Animal Studies at Sanofi approved the protocol for animal experimentation. This protocol and all laboratory procedures comply with the French Legislation, which implements the European Directives. Animals were received at least one week before the experiment to allow acclimatization. The animals were kept under a natural daylight cycle and given food and water *ad libitum.*

All tumor cell lines were obtained from DSMZ GmbH. Initially, cell lines were cultured in RPMI 1640 containing 10% FCS and antibiotics, and implanted subcutaneously in SCID mice ($10^7$ cells/mouse). When the tumor reached approximately 1000 mg, it was removed from the donor mouse, cut into fragments (2-3 mm diameter), placed in a phosphate buffer saline, and implanted bilaterally with a 12-gauge trocar. Tumor fragments were propagated until stable growth behavior occurred (a stable doubling time), before using in experiments. Distribution was performed using body weight and tumor weight criteria with the "Newlab oncology" software (Newlab).

Changes from baseline in tumor volume were used to calculate the median values in treated (ΔT) and control (ΔC) groups. ΔT/ΔC (%) is the ratio of median at any chosen day (the last day before control mice are sacrificed owing to tumor size). ΔT/ΔC values can be translated to activity ratings: ΔT/ΔC <0: highly active, ΔT/ΔC <10%: very active, 10% <ΔT/ΔC <40%: active, ΔT/ΔC >40%: inactive. When ΔT/ΔC values are negative, the percentage of regression is evaluated. Partial regression (PR) is defined as a decrease ≥ 50% of tumor volume at treatment initiation. Complete regression (CR) is defined as a decrease in tumor volume below the limit of palpation (T=10mm$^3$). At study end, the number of tumor-free survivors (TFS), which correspond to mice without any detectable tumor, was determined. Both drug-related deaths and maximum percent relative mean net body weight loss were also determined. Median times to reach tumor target size were compared using Log-Rank or Kruskal-Wallis multiple comparisons tests. A body weight loss nadir (mean of group) > 20% or 10% drug-related deaths were considered to indicate an excessive toxic dosage.

**Results**

**Effect of COMPOUND (A) on normal and AML myeloid progenitors**

The anti-proliferative activity of COMPOUND (A) was evaluated in 5 granulomonocytic and erythroblastic normal myeloid progenitors. The granulomonocytic progenitors (CFU-GM) were more resistant to COMPOUND (A) than leukemic progenitors with median $IC_{50}$: 788.1 nM, interquartile range: 463.5 nM, p = 0.0008, Mann Whitney test). In contrast, erythroblastic BFU-E progenitors were not significantly sensitive to COMPOUND (A) treatment than CFU-L cells with median $IC_{50}$: 218.8 nM (interquartile range: 225.3 nM, p = 0.3142, Mann Whitney test for unpaired data).

The anti-proliferative effect of COMPOUND (A) was also evaluated on the myeloid progenitors (CFU-L) of 29 AML patient samples using a clonogenic test (see Table 1 below). COMPOUND (A) potently inhibited colony formation in the majority of the tested samples, with $IC_{50}$ values ranging from 3.8 to >1000 nM. According to COMPOUND (A) activity ranges on normal hematopoietic progenitors, leukemic samples were then classified as resistant or sensitive: resistant (N=4 samples, $IC_{50}$ minimum: 887.9 nM, maximum: >1000 nM), or sensitive (N = 26, $IC_{50}$ median 171.7 nM, minimum: 3.8 nM, maximum: 531.6 nM) which represents 86.7% of the tested samples (see Table 1 below). In the high-risk cytogenetic group (i.e., patients 4, 11, 14, 19, 21, 23, 25, 27, 28 and 29), only 3 AML cell samples displayed resistance to treatment with COMPOUND (A) (patients 27, 28, 29). However, it was noteworthy that patient sample 11, harbouring a complex karyotype, was highly sensitive to treatment with COMPOUND (A).

Table 1: Evaluation of Compound (A) on the leukemia progenitors (CFU-L) of 30 AML patient samples using an ex vivo clonogenic test.

| Patients number | Age | FAB | Immunologic phenotype | Karyotypic analysis | FLT3/c-kit | IC50 |
|---|---|---|---|---|---|---|
| | | | | | | CFU-L (nM) |
| 1 | 73 | 2 | CD34-CD33- | **46XX** | 0 | 3.8 |
| 2 | 76 | 2 | CD34+CD33+ | **47XX,+11** | 1 | 4.6 |
| 3 | 33 | 2 | CD34+CD33+ | **46XY+X,t(8;21)** | 1* | 18.6 |
| 4 | 19 | 5 | CD34+CD33+ | **complex** | 0 | 26.51 |

(continued)

| Patients number | Age | FAB | Immunologic phenotype | Karyotypic analysis | FLT3/c-kit | IC50 CFU-L (nM) |
|---|---|---|---|---|---|---|
| 5 | 37 | 2 | CD34+CD33+ | **46XX** | 1 | 37.17 |
| 6 | 18 | 2 | CD34-CD33+ | **46XX** | 1 | 52.6 |
| 7 | 81 | 2 | CD34+CD33+ | **46XY** | 1 | 56.9 |
| 8 | 58 | 4 | CD34+CD33+ | **46XY** | 0 | 79.49 |
| 9 | 69 | 2 | CD34+CD33+ | **46XY** | 0 | 81.1 |
| 10 | 72 | 1 | CD34+CD33+ | **46XY, t(1;8)** | 1 | 126.9 |
| 11 | 38 | 1 | CD34+CD33+ | **46XY, t(9;11)** | 0 | 160.7 |
| 12 | 80 | 2 | CD34-CD33+ | **46XX** | Not done | 162.6 |
| 13 | 73 | 1 | CD34+CD33+ | **46XX** | 0 | 167.9 |
| 14 | 70 | 1 | CD34+CD33+ | **46XY, del 7q** | 0 | 175.5 |
| 15 | 42 | 2 | CD34+CD33- | **46XY** | 0 | 213.1 |
| 16 | 40 | 5 | CD34-CD33+ | **46XY** | 1 | 257.4 |
| 17 | 52 | 1 | CD34-CD33+ | **46XX** | 1 | 295.2 |
| 18 | 78 | 5 | CD34+CD33+ | **47XY, +8** | 0 | 299.1 |
| 19 | 63 | 4 | CD34+CD33+ | **46XY,del7q-;1q** | 0 | 302.9 |
| 20 | 15 | 2 | CD34+CD33+ | **46XY, t(8;21)** | 0* | 303.7 |
| 21 | 52 | 5 | CD34+CD33+ | **46XY, t(3;6)** | 1 | 322.2 |
| 22 | 61 | 2 | CD34+CD33+ | **46XY** | 1 | 343.5 |
| 23 | 72 | 1 | CD34+CD33- | **46XX, 7q-** | 0 | 407.6 |
| 24 | 38 | 2 | CD34+CD33+ | **46XX** | 0 | 453.3 |
| 25 | 80 | 2 | Not done | **46XY** | Not done | 531.6 |
| 26 | 47 | 4 | Not done | **46XX** | 1 | 887.9 |
| 27 | 9 | 5 | CD34-CD33+ | **46XX, t(1;11) mll rear** | Not done | >1000 |
| 28 | 55 | 1 | CD34+CD33+ | **Complex** | 0 | >1000 |
| 29 | 70 | 1 | CD34+CD33+ | **Complex** | 0 | >1000 |

**Abreviations and annotations used:** Age at D : age at diagnosis; FAB : French American British classification of leukemias; WBC : White Blood Count at diagnosis; FLT3: FLT3 receptor mutation, i.e : 0: wild type receptor, 1: ITD : Internal Tandem Duplication mutation or D835 kinase domain mutation , or both mutations (ITD and kinase domain) have been screened in all the tested samples; * detection of an activating mutation of c-kit receptor (D688).

***In vivo* effects of compound (A) in mice implanted with KG1 AML cells**

Mice bearing KG1a cells were treated with IV COMPOUND (A) 10 or 17 mg/kg daily from day 5 to day 13, and with IP COMPOUND (A) on days 15, 16, 18-20, 22, 24-26, 29 and 31. A 16.2% body weight loss was seen at nadir (day 22) with COMPOUND (A) 17 mg/kg, which was reversible after treatment was stopped. At 10 and 17 mg/kg, COMPOUND (A) was highly active: ΔT/ΔC evaluated on day 32 was negative at both dosages (-4.99 [range: -6.46, -3.66] and —4.70 [range: -6.90, -3.91], respectively). At both dosages, 10/10 animals experienced complete regression with 80% and 60% cures observed, respectively, on day 120 at these two dosages. Statistical analysis confirmed the activity of COMPOUND (A) at both dosages based on the tumor volume and on the time for tumors to reach 1000 mg.

Mice bearing KG1 cells were treated daily with IV COMPOUND (A) from day 19 to day 38 post-tumor inoculations

at 10 and 25 mg/kg/day. At 25 mg/kg/day, the DT/DC was inferior to -14.27 (range: -29.81, -7.12). Body weight loss amounting to 18.2% was observed on day 29 at the end of the treatment period. Weight loss was reversible, indicating that 25mg/kg was the MTD. A total of 6 out of 6 animals experienced complete regressions (CR) and cures at MTD. At 10mg/kg/day, ΔT/ΔC was 17.45 (range: -0.52, 52.10), and one of six animals experienced CR. This indicated that COMPOUND (A) was active at 10 mg/kg, and highly active at 25 mg/kg. Statistical analysis confirmed the anti-tumor activity of COMPOUND (A) at these two dosages.

The anti-tumor activity of COMPOUND (A) was also evaluated in mice bearing KG1 cells when given by oral route. When two daily dosages of 40 mg/kg/day were given consecutively from day 15 to day 40, post-tumor inoculation, ΔT/ΔC was -3.80 (range: -5.21, -2.93). A maximum body weight loss of 14.4% was detected on day 28, which was rapidly reversible. None of the animals were cured but 7 animals out of 10 experienced a CR. When given orally once a day at 50 or 31.5 mg/kg, the DT/DC was 7.61 (range: 4.72, 12.88) or 19.72 (range: 15.81, 29.35) suggesting efficacy. However, at 19.8mg/kg, a ΔT/ΔC of 41.99 (range: 32.19, 59.33) was observed, suggesting that there is no efficacy at this dose.

One aim was to generate an inhibitor of AML cells, which could be administered by IV and oral routes. The anti-tumor activity of COMPOUND (A) when dosed as a single agent, at a clinically relevant induction/consolidation regimen, was evaluated in very advanced KG1 bearing mice (1000 mg tumor size at the onset of treatment). COMPOUND (A) was first administered daily at 25 mg/kg IV from day 22 until day 30 post-tumor inoculation. Then the treatment was switched to daily oral dosing at 50 mg/kg or 2 x 40mg/kg as previously tested from day 31 to day 95. During the IV induction phase, COMPOUND (A) dosed at 25 mg/kg was highly active and induced strong tumor regressions. At day 31, mice were treated daily with oral dosages of COMPOUND (A) at either 2 x 40 mg/kg or 1 x 50 mg/kg. While an immediate re-growth of tumors was observed in the vehicle group, in mice treated with COMPOUND (A) at 1 x 50 mg/kg, tumors re-grew slowly, and at the 2 x 40 mg/kg oral dosage, complete disappearance of tumors was noted. This experiment suggests that following an IV induction phase; COMPOUND (A) could be successfully given orally to mimic a consolidation phase in the clinic as a single agent.

**Combination of cytarabine and compound (A) was evaluated in mice bearing KG1 cells (Figure 1).**

Compound (A) 20 mg/kg was administered on days 16 to 20 by IV route and Cytarabine 31.5 mg/kg was administered by IP route on days 16 to 22.

Single agent cytarabine showed log cell kill-gross (LCK-g) of 1.6 with no CR, PR or cure. Compound (A) showed LCK-g of 2.4 with 83% CR and no cure. Combination of the two products elicited significant synergy: LCK-g of 5.9 with 100% CR and 66% cures (see figure 1). Combination of cytarabine and compound (A) also showed synergistic anti-tumor activity in mice implanted with Kasumi-1 and CML-T1 tumor cells.

<u>**EXAMPLE 2 : Activity on CML**</u>

The effect of Compound (A) injected IP with cytarabine on the development of the human chronic leukaemia CML-T1 implanted in mice was assessed as follows.

**Materials and methods**

Eight- week- old female SCID mice were purchased from Charles River (L'arbresle, Lyon, France) . All animals rested for 7 days prior to the onset of treatments, and animal protocols were approved by the Animal Studies Committee of Sanofi Aventis Recherche & Développement. This protocol and the laboratory procedures comply with French legislation, which implements the European Directives. Animals were received at least one week before the experiment, to allow a perfect acclimatization. Animal health was examined at the day before tumor implantation and before randomization to ensure that only animals of good health were selected to enter in the testing procedures. They were housed in macrolon type III cages with filter hoods in a sterile room in which the air is continuously filtered to avoid any contamination. The sterility of the room is checked once a month and the cages were sterilized at 121 °C for 30 minutes before use and changed twice a week. Room temperature was maintained at 22°C, and relative humidity at 60±10%. The animals were kept under a natural daylight cycle. The animals were fed with R03 irradiated at 10 kGy, purchased from UAR (91360 Epinay/ Orge, France) , and water sterilized at 121 °C for 30 minutes. Water consumption was visually monitored daily and the bottles were changed twice a week. Food and water were given *ad libitum.* The animal bedding was produced by UAR and sterilized at 121 °C for 30 minutes and renewed twice a week.

Compound (A) was prepared by mixing 5% DMSO with 10% Tween-80 and 85% H2O. Cytarabine (B) was prepared in water for injectable preparation (Aracytine®, also referred to as Ara-C).

Solutions were kept on ice and administered as a bolus within 1 hour after formulation. The volume of injection per mouse was 0.2 mL.

Animals were treated by intraperitoneal (IP) route on days 8, 9, 11 to 17 and 19 with COMPOUND (A) and on

days 8, 12 and 16 with Cytarabine.

**Tumor information and implantation**

CML- T1 is a human T cell leukemia established from the peripheral blood of a 36- year- old woman with CML in blast crisis in 1987. Cells were described to express T cell surface markers and to have a Bcr- abl translocation (producing the p210 Bcr- abl protein) (Kuriyama et al Blood. 1989; 74 (4) : 1381- 7) . The immunology of the tumor is the following: CD2-, smCD3 (+) , cyCD3 +, CD4 +, CD5 +, CD6 +, CD7 +, CD8 +, CD13-, CD19-, CD34-, TCRalpha/ beta-, TCRgamma/ delta.

The techniques of chemotherapy and data analysis have been presented in details (Corbett et al., Invest. New Drugs 1998;16:129-39). Initially, this cell line was cultured in RPMI 1640 containing 10% foetal calf serum (FCS) and antibiotics, and implanted subcutaneously in SCID mice ($10^7$ cells/mouse). When the tumor reached approximately 1000 mg, it was removed from the donor mice, cutted into fragments (2-3 mm diameter), placed in a phosphate buffer saline, and implanted bilaterally with a 12 gauge trocar. Tumor fragments were propagated until stable growth behavior occurred a stable doubling time (td), before using in experiments. Tumors fragments were frozen with 80% medium, 10% foetal calf serum (FCS), 10% DMSO at 6-10 fragments/vial.

**Grouping identification and randomization of animals**

The mice were within a 19-20 g weight range. Animals with a body weight inferior to 18 g were excluded of the study. On day 8, tumor bearing animals were stratified into several groups. Only animals with 2 appropriate tumor volumes were selected and randomly distributed to treatment and control groups. The average tumor weight at start of therapy was 63-77 mg. Distribution was performed using body weight and tumor weight criteria with the "Newlab oncology" software (Newlab, 23 bd Europe, 54500 Vandoeuvre les Nancy, France). Each group consisted of 6-7 mice. At the beginning of the study, each cage was labelled with a record card, indicating the date of tumor implantation, tumor type, test compound and route of administration.

**Criteria for assessing antitumor activity**

Chemotherapy was started on the day of grouping (8 days after tumor implantation). Mice were checked daily and adverse clinical reactions noted. Each parameter was measured and results recorded using the "Newlab oncology" software.

***Tumor Weights***

Tumors were measured with a caliper twice weekly until the tumor reached 2000 mg or until the animal died (which ever come first). Tumor weights were estimated from 2 dimensional measurements: Weight (in mg) = (a x $b^2$)/2, where "a" and "b" are the tumor length and width (mm) respectively. The total weight of the 2 implanted tumors is indicated.

Mice with complete regression (CR) consist in tumor regression below limit of palpation (<63 mg). At the end of the study, the number of tumor free survivors (TFS), which correspond to mice without tumor weight superior to 63 mg, was determined.

***Determination of the tumor doubling time***

The tumor doubling time (Td) is estimated in the control group, with the estimated slope "a" of the linear model of the log tumoral weight along day chosen in the exponential growth phase (100 to 1000 mg range), with Td = log2/a.

***Quantification of tumor cell kill***

For subcutaneous (SC) growing tumors, the total log cell kill-gross (LCK-g) is calculated from the following formula (Corbett et al., Invest. New Drugs 1998;16:129-39.):

$$\text{The LCK-g} = \frac{\text{(T-C) value in days}}{3.32 \times \text{Td}}$$

where T is the median time (in days) required for the treatment group tumors to reach a predetermined size (eg, 1000

mg), and C is the median time tumors to reach the same size (in days) for the vehicle group of each schedule. Tumor-free survivors are excluded from these calculations (cures are tabulated separately). T-C is the tumor growth delay and Td is the tumor doubling time in days. The conversion of the T-C values to LCK-g is possible because the Td of tumors regrowing post treatment approximates the Td values of the tumors in untreated control mice. LCK-g values can be translated into an activity rating, according to the Southern Research Institute (SRI) criteria:

| SRI activity criteria | LCK gross (Treatment duration of 5-20 days) |
|---|---|
| Highly active ++++ | >2.8 |
| +++ | 2.0 to 2.8 |
| ++ | 1.3 to 1.9 |
| + Inactive | 0.7 to 1.2 |
| - | < 0.7 |

The second endpoint used to assess antitumor activity was the evaluation of the T/C. The T/C value in percent is an indication of antitumor effectiveness. The treatment and control groups are measured when the control group tumors reach approximately 700 to 1200 mg in size. A T/C equal to or less than 42% is considered significant antitumor activity by the Drug Evaluation Branch of the Division of Cancer Treatment of the National Cancer Institute.

***Drug toxicity***

A body weight loss nadir (mean of group) of greater than 20% or 10% drug deaths are considered to indicate an excessively toxic dosage. Antitumor activity evaluation was done at the highest non toxic dose (HNTD).

***Statistical analysis***

In order to evaluate the compounds and association effects, a 3 way ANOVA with repeated measures on factor day was applied on log tumoral weight (until the maximum time measurement of vehicle group), followed by a Dunnett's adjustment for multiplicity. Additional statistical analysis was done to evaluate synergism between the 2 products (COMPOUND (A) and Cytarabine) at fixed day, the association effect is compared to the sum of the effects of the compound alone at the defined doses. All Statistical analyses were performed on SAS V8.2 for Windows software.
A probability less than 5% ($p<0.05$) was considered as significant. The statistical analyses are included in an independent statistical report (TL06010- EN- E01) .

**<u>RESULTS</u>**

The activity of COMPOUND (A) was determined in CML-T1 bearing mice. A control group (no treatment) and a vehicle-treated group are included in the study. These are made up of mice carrying tumors either untreated or treated with the vehicle. The control group serves to study the effect of the vehicle and the vehicle-treated group serves as reference to the different treatments.

**Cytarabine treated groups :**

Cytarabine was administered by IP route on days 8, 12 and 16 at 5 dosages (50, 100, 150, 200, 250 mg/kg).

**COMPOUND (A) treated groups:**

COMPOUND (A) was administered by IP route on days 8, 9, 11 to 17 and 19 at 4 dosages (10, 17, 25 or 30 mg/kg/administration).

**Cytarabine + COMPOUND (A) treated groups:**

COMPOUND (A) and Cytarabine were administered by IP route respectively on days 8, 9, 11 to 17 and 19 for COMPOUND (A) and on days 8, 12 and 16 for Cytarabine.
Cytarabine 200 mg/kg (total dose = 600/100 mg/kg): a 4.4% body weight loss was observed on day 21 and no drug death was detected. LCK-g = 1.9 with 1 out of 7 animals considered as cured on day 141 at the end of the study.

Compared to the different compounds at their respective doses, Cytarabine at 200 mg/kg produced only a 0.9 LCK-g and COMPOUND (A) at 10 mg/kg produced a 0.1 LCK-g. This indicated that this combination is active, producing a higher LCK-g than the isolated compounds at their respective doses. Statistical analyzes detected a synergistic effect on day 20 only of the experiment.

Results are illustrated on Figure 2.

**CONCLUSION**

Synergism has been evaluated on log tumoral weight at fixed day for a combination of dose of the 2 products by comparison of the sum of the effect of each product alone at these doses and the effect of this combination of the 2 products, compared with the vehicle Cytarabine + vehicle Compound (A) group. There is a significant synergism on day 20 between COMPOUND (A) at 10 mg/kg and Cytarabine at 200 mg/kg. The synergism becomes significant between all Cytarabine doses and COMPOUND (A) at 17 mg/kg on day 26.

**Claims**

1. A combination comprising the compound N-[2-(2,1,3-benzothiadiazol-5-ylamino)-6-(2,6-dichlorophenyl)pyrido[2,3-d]pyrimidin-7-yl]-N'-(1,1-dimethylethyl)-urea (A) or a hydrate, a salt or a solvate thereof, with cytarabine (B) for use in the treatment of acute myeloid leukaemias.

2. A combination comprising the compound N-[2-(2,1,3-benzothiadiazol-5-ylamino)-6-(2,6-dichlorophenyl)pyrido[2,3-d]pyrimidin-7-yl]-N'-(1,1-dimethylethyl)-urea (A) or a hydrate, a salt or a solvate thereof, with cytarabine (B) for use in the treatment of chronic myeloid leukaemias.

3. A combination for use according to claim 1 or 2, wherein compound (A) is administered by the intravenous route.

4. A combination for use according to claim 1 or 2, wherein compound (A) is administered by the oral route.

5. A combination for use according to claim 1 or 2, wherein compound (A) is administered by the intravenous route followed by the oral route.

6. A combination for use according to claim 1 or 2, wherein compound (A) is administered by the intraperitoneal route.

7. A combination for use according to anyone of the preceding claims, wherein cytarabine (B) is administered by the intravenous route.

8. A combination for use according to anyone of the preceding claims, wherein cytarabine (B) is administered by the intraperitoneal route

9. A combination for use according to claim 1, wherein compound (A) is administered by the intravenous route and cytarabine (B) is administered by the intravenous route.

10. A combination for use according to claim 1, wherein compound (A) is administered by the oral route and cytarabine (B) is administered by the intravenous route.

11. A combination for use according to claim 1, wherein compound (A) is administered by the intravenous route followed by the oral route and cytarabine (B) is administered by the intravenous route.

12. A combination for use according to claim 2, wherein compound (A) is administered by the intravenous route and cytarabine (B) is administered by the intravenous route.

13. A combination for use according to claim 2, wherein compound (A) is administered by the oral route and cytarabine (B) is administered by the intravenous route.

14. A combination for use according to claim 2, wherein compound (A) is administered by the intravenous route followed by the oral route and cytarabine (B) is administered by the intravenous route.

**15.** A combination for use according to claim 2, wherein compound (A) is administered by the intraperitoneal route followed by the oral route and cytarabine (B) is administered by the intraperitoneal route.

**16.** A combination for use according to anyone of the previous claims wherein compounds (A) and (B) are administered simultaneously, separately or sequentially.

**17.** A combination for use according to anyone of the preceding claims, where it is for treating AML or CML patients resistant to standard chemotherapy.

**18.** A combination for use according to anyone of the preceding claims, where it is for treating AML or CML in high risk cytogenetic patients.

**19.** A kit comprising :

- the compound N-[2-(2,1,3-benzothiadiazol-5-ylamino)-6-(2,6-dichlorophenyl) pyrido[2,3-d]pyrimidin-7-yl]-N'-(1,1-dimethylethyl)-urea (A) or a hydrate, a salt or a solvate thereof,
- cytarabine (B)
- instructions for use in the treatment of acute myeloid leukaemias.

**20.** A kit comprising :

- the compound N-[2-(2,1,3-benzothiadiazol-5-ylamino)-6-(2,6-dichlorophenyl) pyrido[2,3-d]pyrimidin-7-yl]-N'-(1,1-dimethylethyl)-urea (A) or a hydrate, a salt or a solvate thereof,
- cytarabine (B)
- instructions for use in the treatment of chronic myeloid leukaemias.

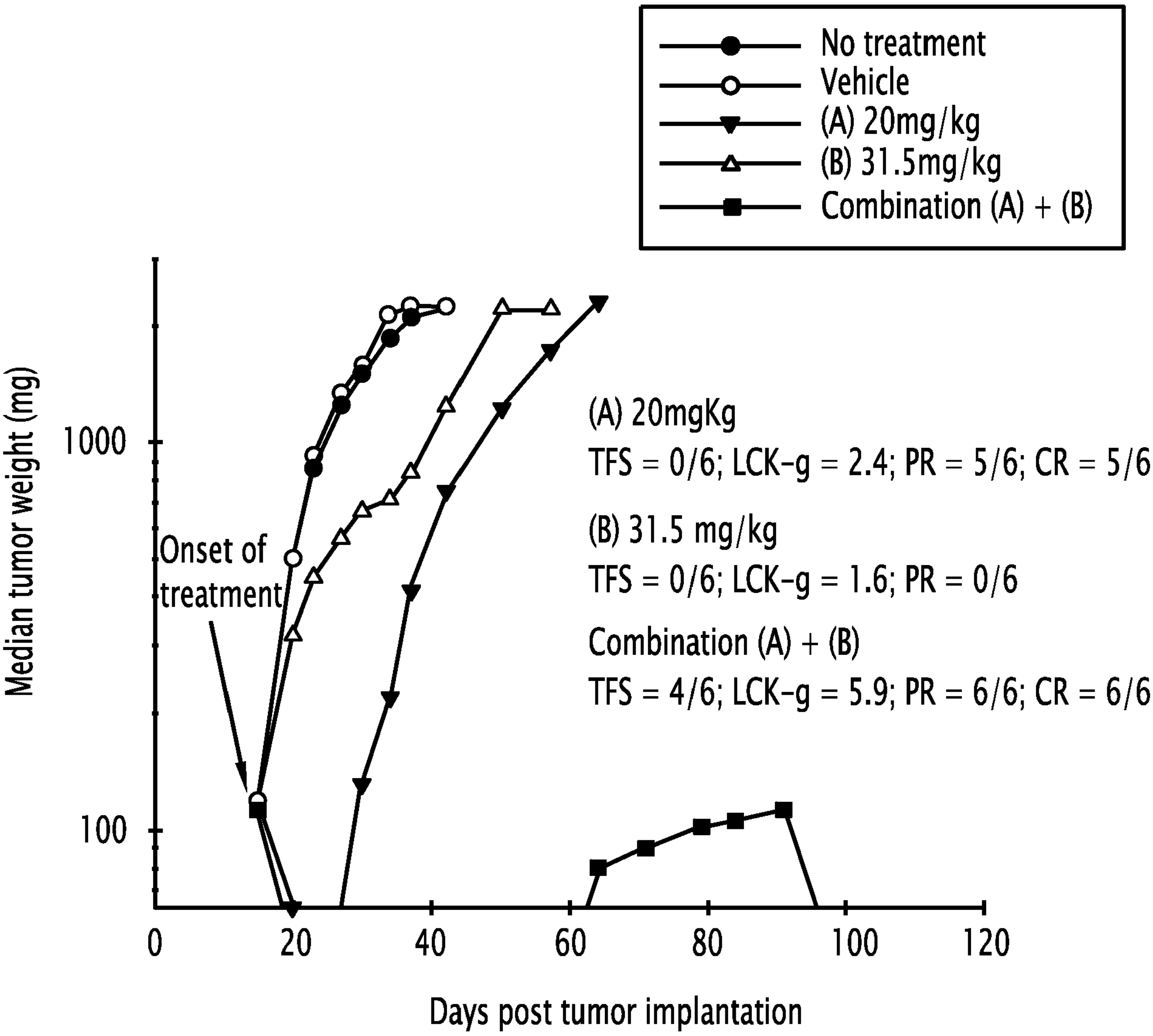

No treatment
Vehicle
(A) 20mg/kg
(B) 31.5mg/kg
Combination (A) + (B)
(A) 20mgKg
TFS = 0/6; LCK-g = 2.4; PR = 5/6; CR = 5/6
(B) 31.5 mg/kg
TFS = 0/6; LCK-g = 1.6; PR = 0/6
Combination (A) + (B)
TFS = 4/6; LCK-g = 5.9; PR = 6/6; CR = 6/6
Onset of treatment
Median tumor weight (mg)
1000
100
0
20
40
60
80
100
120
Days post tumor implantation


FIG.1

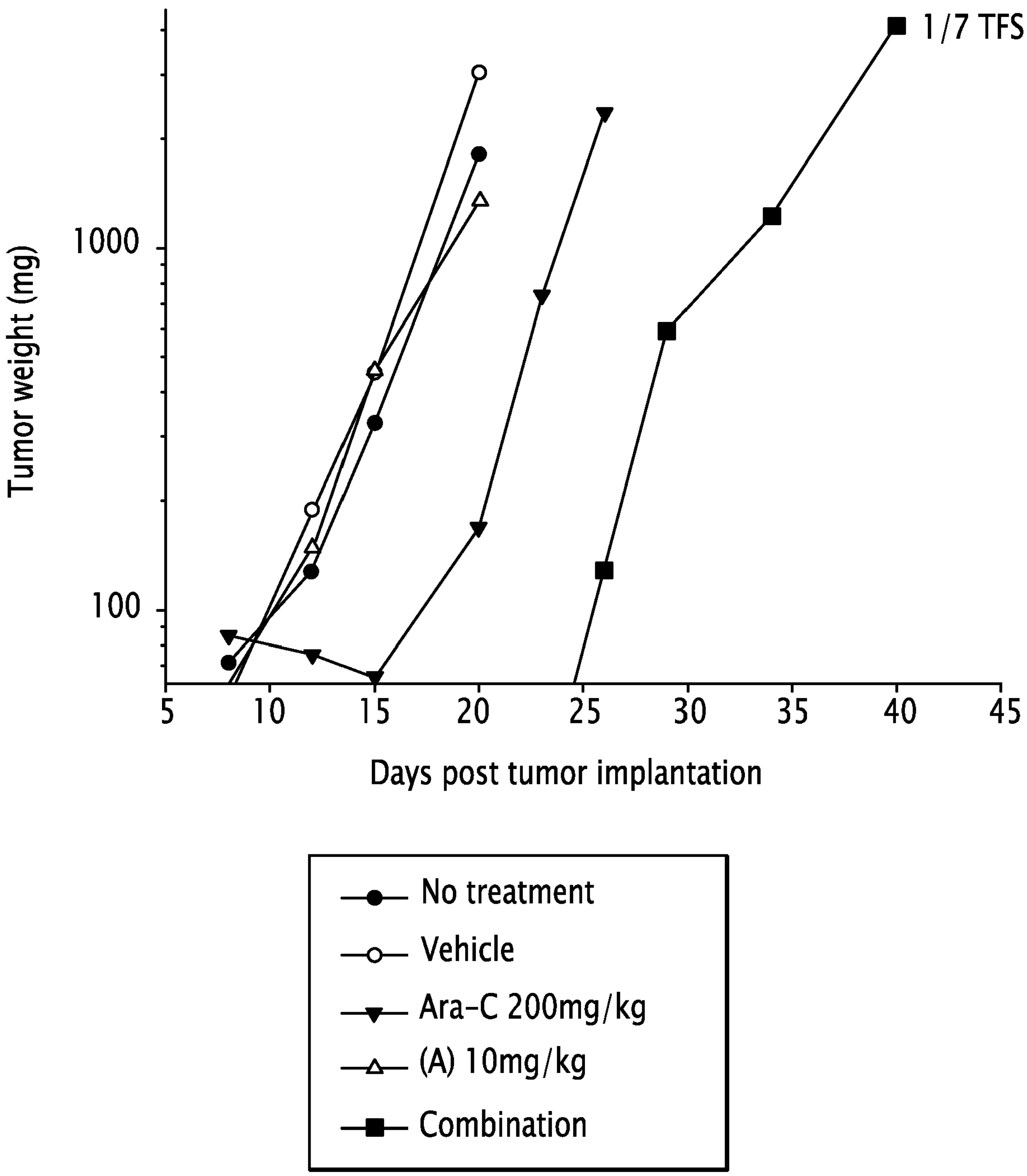
1/7 TFS
1000
100
Tumor weight (mg)
5
10
15
20
25
30
35
40
45
Days post tumor implantation
No treatment
Vehicle
Ara-C 200mg/kg
(A) 10mg/kg
Combination

FIG.2

Europäisches Patentamt
European Patent Office
Office européen des brevets

# EUROPEAN SEARCH REPORT

Application Number
EP 12 30 5295

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/298790 A1 (BOURRIE BERNARD [FR] ET AL) 3 December 2009 (2009-12-03)<br>* paragraph [0061] *<br>* claims 1, 3, 15, 16 *<br>----- | 1-20 | INV.<br>A61K31/704<br>A61K31/7068 |
| A | US 2008/176874 A1 (BOURRIE BERNARD [FR] ET AL) 24 July 2008 (2008-07-24)<br>* paragraph [0129] *<br>* claims 14,17 *<br>----- | 1-20 | |
| A | MARLEY S B ET AL: "EFFECTS OF COMBINATIONS OF THERAPEUTIC AGENTS ON THE PROLIFERATION OF PROGENITOR CELLS IN CHORNIC MYELOID LEUKAEMIA",<br>BRITISH JOURNAL OF HAEMATOLOGY, WILEY-BLACKWELL PUBLISHING LTD, GB,<br>vol. 116, no. 1,<br>1 January 2002 (2002-01-01), pages 162-165, XP008016852,<br>ISSN: 0007-1048, DOI: 10.1046/J.1365-2141.2002.03237.X<br>* the whole document *<br>----- | 1-20 | TECHNICAL FIELDS SEARCHED (IPC) |
| A | RITTER J: "Acute myeloid leukaemias",<br>EUROPEAN JOURNAL OF CANCER, PERGAMON PRESS, OXFORD, GB,<br>vol. 34, no. 6, 1 May 1998 (1998-05-01), pages 862-872, XP004284916,<br>ISSN: 0959-8049, DOI: 10.1016/S0959-8049(98)00132-4<br>* the whole document *<br>-----<br>-/-- | 1-20 | A61K |

The present search report has been drawn up for all claims

1

| Place of search | Date of completion of the search | Examiner |
|---|---|---|
| Munich | 27 June 2012 | Werner, Doris |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 30 5295

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | TALLMAN MARTIN S ET AL: "Drug therapy for acute myeloid leukemia", BLOOD, AMERICAN SOCIETY OF HEMATOLOGY, US, vol. 106, no. 4, 15 August 2005 (2005-08-15), pages 1154-1163, XP002572671, ISSN: 0006-4971, DOI: 10.1182/BLOOD-2005-01-0178 * the whole document * ----- | 1-20 | |
| | | | TECHNICAL FIELDS SEARCHED (IPC) |

The present search report has been drawn up for all claims

1

| Place of search | Date of completion of the search | Examiner |
|---|---|---|
| Munich | 27 June 2012 | Werner, Doris |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

**ANNEX TO THE EUROPEAN SEARCH REPORT ON EUROPEAN PATENT APPLICATION NO.** EP 12 30 5295

This annex lists the patent family members relating to the patent documents cited in the above-mentioned European search report.
The members are as contained in the European Patent Office EDP file on
The European Patent Office is in no way liable for these particulars which are merely given for the purpose of information.

27-06-2012

| Patent document cited in search report | Publication date | Patent family member(s) | Publication date |
|---|---|---|---|
| US 2009298790 A1 | 03-12-2009 | AR 064550 A1 | 08-04-2009 |
| | | AT 534392 T | 15-12-2011 |
| | | AU 2007347370 A1 | 28-08-2008 |
| | | CA 2672716 A1 | 28-08-2008 |
| | | CN 101568338 A | 28-10-2009 |
| | | CO 6190610 A2 | 19-08-2010 |
| | | CR 10881 A | 12-08-2009 |
| | | DK 2107906 T3 | 19-03-2012 |
| | | EA 200970639 A1 | 30-04-2010 |
| | | EC SP099449 A | 31-07-2009 |
| | | EP 2107906 A2 | 14-10-2009 |
| | | ES 2377843 T3 | 02-04-2012 |
| | | FR 2910813 A1 | 04-07-2008 |
| | | HR 20120170 T1 | 31-03-2012 |
| | | JP 2010514743 A | 06-05-2010 |
| | | MA 31100 B1 | 04-01-2010 |
| | | NZ 577991 A | 12-01-2012 |
| | | PE 15482008 A1 | 04-01-2009 |
| | | PT 2107906 E | 05-03-2012 |
| | | SI 2107906 T1 | 30-03-2012 |
| | | SV 2009003315 A | 05-02-2010 |
| | | TW 200833343 A | 16-08-2008 |
| | | US 2009298790 A1 | 03-12-2009 |
| | | WO 2008102075 A2 | 28-08-2008 |
| | | ZA 200904478 A | 25-08-2010 |
| US 2008176874 A1 | 24-07-2008 | AR 054812 A1 | 18-07-2007 |
| | | AT 423119 T | 15-03-2009 |
| | | AU 2006264801 A1 | 11-01-2007 |
| | | BR PI0613020 A2 | 14-12-2010 |
| | | CA 2610794 A1 | 11-01-2007 |
| | | CN 101213196 A | 02-07-2008 |
| | | CN 102260258 A | 30-11-2011 |
| | | CR 9568 A | 20-02-2008 |
| | | DK 1902054 T3 | 15-06-2009 |
| | | DO P2006000145 A | 31-01-2008 |
| | | EA 200800223 A1 | 30-06-2008 |
| | | EC SP078011 A | 23-01-2008 |
| | | EP 1902054 A1 | 26-03-2008 |
| | | EP 2020410 A1 | 04-02-2009 |
| | | ES 2322101 T3 | 16-06-2009 |
| | | FR 2887882 A1 | 05-01-2007 |
| | | HR 20090236 T3 | 31-05-2009 |
| | | JP 4472009 B2 | 02-06-2010 |
| | | JP 2008544975 A | 11-12-2008 |
| | | KR 20080021714 A | 07-03-2008 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 12 30 5295

This annex lists the patent family members relating to the patent documents cited in the above-mentioned European search report.
The members are as contained in the European Patent Office EDP file on
The European Patent Office is in no way liable for these particulars which are merely given for the purpose of information.

27-06-2012

| Patent document cited in search report | Publication date | Patent family member(s) | Publication date |
|---|---|---|---|
| | | MA 29561 B1 | 02-06-2008 |
| | | MY 143089 A | 15-03-2011 |
| | | NZ 564072 A | 24-02-2012 |
| | | PA 8684801 A1 | 17-01-2007 |
| | | PE 01392007 A1 | 07-03-2007 |
| | | PT 1902054 E | 22-04-2009 |
| | | SI 1902054 T1 | 30-06-2009 |
| | | TW I321566 B | 11-03-2010 |
| | | US 2008176874 A1 | 24-07-2008 |
| | | US 2009163522 A1 | 25-06-2009 |
| | | UY 29645 A1 | 31-01-2007 |
| | | WO 2007003765 A1 | 11-01-2007 |
| | | ZA 200711032 A | 29-04-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007003765 A
- WO 2008102075 A


### Non-patent literature cited in the description


- Cancer Facts and Figures. American Cancer Society, 2006
- **BENNETT et al.** Proposals for the classification of the acute leukaemias. French-American-British (FAB) co-operative group. *Br J Haematol,* 1976, vol. 33 (4), 451-8
- **PLO et al.** *Mol Pharmacol,* 2002, vol. 62, 304-312
- *Cancer Res.,* 01 October 1988, vol. 48 (19), 5447-54
- *Cancer Chemother Pharmacol.,* 1996, vol. 38, 548-552
- Remington's Pharmaceutical Sciences. Mack Publishing Company, 1985
- **S. M. BERGE et al.** Pharmaceutical Salts. *J. Pharm. Sci.,* 1977, vol. 66, 1-19
- Remington's Pharmaceutical Sciences. Mack Publishing Company, 1990
- **MIZUKI M. et al.** *Blood,* 2000, vol. 96, 3907-3914
- **FIEBIG H.H. et al.** *Eur. J. Cancer,* 2004, vol. 40, 802-820
- **BONNET D. et al.** *Nat Med,* 1997, vol. 3, 730-737
- **RÉCHER C. et al.** *Blood,* 2005, vol. 105, 2527-2534
- **KURIYAMA et al.** *Blood.,* 1989, vol. 74 (4), 1381-7
- **CORBETT et al.** *Invest. New Drugs,* 1998, vol. 16, 129-39